# EUROPEAN PATENT SPECIFICATION

(11) **EP 3 957 732 B1**
(45) Date of publication and mention of the grant of the patent: **24.06.2026**
(21) Application number: 20791329.4
(22) Date of filing: 19.03.2020
(51) Int. Cl.: C12N 15/09, C12Q 1/6869, C12Q 1/6886

(54) **METHOD AND KIT FOR DETECTING RISK OF COLORECTAL CANCER**
VERFAHREN UND KIT ZUR ERKENNUNG DES RISIKOS FÜR EIN KOLOREKTALKARZINOM
PROCÉDÉ ET KIT DE DÉTECTION DU RISQUE DE CANCER COLORECTAL

(30) Priority: 18.04.2019 JP 2019079535
(43) Date of publication of application: 23.02.2022
(73) Proprietor: Keio University, Tokyo, 108-8345 (JP)
(72) Inventor: SATO, Toshiro, Tokyo 160-8582 (JP)
(74) Representative: HGF
(86) International application number: PCT/JP2020/012468
(87) International publication number: WO 2020/213344

(56) References cited:
- US-A1- 2018 051 347
- CHI YAN ET AL: "IL-17R deletion predicts high-grade colorectal cancer and poor clinical outcomes", INTERNATIONAL JOURNAL OF CANCER, vol. 145, no. 2, 28 January 2019 (2019-01-28), US, pages 548 - 558, XP055751269, ISSN: 0020-7136, DOI: 10.1002/ijc.32122
- GRACIELE ALMEIDA DE OLIVEIRA ET AL: "Inducible Nitric Oxide Synthase in the Carcinogenesis of Gastrointestinal Cancers", ANTIOXIDANTS AND REDOX SIGNALING, vol. 26, no. 18, 20 June 2017 (2017-06-20), US, pages 1059 - 1077, XP055751277, ISSN: 1523-0864, DOI: 10.1089/ars.2016.6850
- TRAICOFF JUNE L ET AL: "Characterization of the human polymeric immunoglobulin receptor (PIGR) 3'UTR and differential expression of PIGR mRNA during colon tumorigenesis", JOURNAL OF BIOMEDICAL SCIENCE, KARGER, BASEL, CH, vol. 10, no. 6, 1 November 2003 (2003-11-01), pages 792 - 804, XP009147615, ISSN: 1021-7770
- ESTEBAN-JURADO CLARA ET AL: "Whole-exome sequencing identifies rare pathogenic variants in new predisposition genes for familial colorectal cancer", vol. 17, no. 2, 1 February 2015 (2015-02-01), New York, pages 131 - 142, XP093005629, ISSN: 1098-3600, Retrieved from the Internet <URL:https://www.ncbi.nlm.nih.gov/pmc/articles/PMC4318970/pdf/gim201489a.pdf> DOI: 10.1038/gim.2014.89
- TESTA UGO ET AL: "Colorectal Cancer: Genetic Abnormalities, Tumor Progression, Tumor Heterogeneity, Clonal Evolution and Tumor-Initiating Cells", MEDICAL SCIENCES, vol. 6, no. 2, 13 April 2018 (2018-04-13), pages 31, XP055941696, DOI: 10.3390/medsci6020031
- GRUNDTNER P ET AL: "The IL-10R1 S138G loss-of-function allele and ulcerative colitis", GENES AND IMMUNITY, NATURE PUBLISHING GROUP, GB, vol. 10, no. 1, 18 September 2008 (2008-09-18), pages 84 - 92, XP037768172, ISSN: 1466-4879, [retrieved on 20080918], DOI: 10.1038/GENE.2008.72
- YAN CHI, HUANG WEEI‐YUAN, BOUDREAU JEANETTE, MAYAVANNAN ANIMAMALAR, CHENG ZHENYU, WANG JUN: "IL -17R deletion predicts high-grade colorectal cancer and poor clinical outcomes", INT. J. CANCER, vol. 145, no. 2, 10 January 2019 (2019-01-10), pages 548 - 558, XP055751269, DOI: 10.1002/ijc.32122
- ALMEIDA DE OLIVEIRA, GRACIELE ET AL.: "Inducible Nitric Oxide Synthase in the Carcinogenesis of Gastrointestinal Cancers", ANTIOXIDANTS & REDOX SIGNALING, vol. 26, no. 18, 18 November 2017 (2017-11-18), pages 1059 - 1077, XP055751277, DOI: 10.1089/ars.2016.6850

## Description

### Technical Field

The present invention relates to a method and a kit for determining a risk of colorectal cancer. Priority is claimed on Japanese Patent Application No. 2019-079535, filed April 18, 2019.

### Background Art

Patients with ulcerative colitis are known to have a high risk of cancer. However, reliable markers that can detect a risk of cancer of a patient with ulcerative colitis are not known. For this reason, it is recommended that patients who have had ulcerative colitis for more than 8 years undergo endoscopy every 1 to 2 years. However, endoscopy of a patient with ulcerative colitis needs to be carried out by highly skilled specialists, and the physical burden on the patient is not small. As a result, there is a need for a technique for easily determining the risk of cancer of a patient with ulcerative colitis.

For example, Patent Document 1 describes that galectin-3 and galectin-4 are colorectal cancer markers.

### Citation List

### Patent Document

[Patent Document 1] PCT International Publication No. WO2011/148668

CHI YAN ET AL, INTERNATIONAL JOURNAL OF CANCER, vol. 145, no. 2, 28 January 2019, pages 548-558, relates to IL-17R deletion as predicting high-grade colorectal cancer and poor clinical outcomes.

TRAICOFF JUNE L ET AL, JOURNAL OF BIOMEDICAL SCIENCE, KARGER, BASEL, CH, vol. 10, no. 6, 1 November 2003, pages 792-804, relates to the characterization of the human polymeric immunoglobulin receptor (PIGR) 3'UTR and differential expression of PIGR mRNA during colon tumorigenesis.

ESTEBAN-JURADO CLARA ET AL, GENETICS IN MEDICINE, vol. 17, no. 2 1 February 2015, pages 131-142, relates to Whole-exome sequencing as identifying rare pathogenic variants in new predisposition genes for familial colorectal cancer.

### Summary of Invention

### Technical Problem

An object of the present invention is to provide a technique for determining the risk of colorectal cancer.

### Solution to Problem

The present invention is defined by the appended claims.

Thus, the present invention relates to a method for determining a risk of colorectal cancer of a patient with ulcerative colitis, comprising: a step of detecting a loss-of-function mutation of a gene involved in interleukin (IL)-17 signaling in a colorectal epithelial tissue sample of an inflamed and non-cancer region derived from the patient, wherein the gene involved in IL-17 signaling is a PIGR gene, an NFKB Inhibitor Zeta (NFKBIZ) gene, a TRAF3 Interacting Protein 2 (TRAF3IP2) gene, an Interleukin 17 Receptor A (IL17RA) gene, a Zinc Finger CCCH-Type Containing 12A (ZC3H12A) gene, or a Nitric Oxide Synthase 2 (NOS2) gene, wherein a presence of the loss-of-function mutation indicates that the patient has a high risk of colorectal cancer.

In said method, the step of detecting the loss-of-function mutation of the gene involved in IL-17 signaling can be carried out by immunostaining of a PIGR protein, a secretory IgA protein, an NFKBIZ protein, a TRAF3IP2 protein, an IL17RA protein, a ZC3H12A protein, or a NOS2 protein in the colorectal epithelial tissue sample, and as a result of the immunostaining, a case where a presence of cells in which an expression of the PIGR protein, the secretory IgA protein, the NFKBIZ protein, the TRAF3IP2 protein, the IL17RA protein, the ZC3H12A protein, or the NOS2 protein is not observed is detected in the colorectal epithelial tissue sample indicates that the loss-of-function mutation is present.

Present invention also relates to a kit for determining a risk of colorectal cancer of a patient with ulcerative colitis, consisting of: probes for detecting loss-of-function mutation of a PIGR gene, an NFKBIZ gene, a TRAF3IP2 gene, an IL17RA gene, a ZC3H12A gene, and a NOS2 gene; or primers for amplifying a PIGR gene, an NFKBIZ gene, a TRAF3IP2 gene, an IL17RA gene, a ZC3H12A gene, and a NOS2 gene; or specific binding substances to a PIGR protein, a secretory IgA protein, an NFKBIZ protein, a TRAF3IP2 protein, an IL17RA protein, a ZC3H12A protein, and a NOS2 protein.

### Advantageous Effects of Invention

According to the present invention, it is possible to provide a technique for determining the risk of colorectal cancer.

### Brief Description of Drawings

Fig. 1 is a diagram showing analysis results in Experimental Example 2.
Fig. 2 is a diagram showing analysis results in Experimental Example 2.
Fig. 3 is a diagram showing analysis results in Experimental Example 3.
Figs. 4 (a) to (d) are representative photomicrographs showing results of immunostaining of a colorectal epithelial tissue sample in Experimental Example 4.
Fig. 5 is a diagram showing analysis results in Experimental Example 5.

### Description of Embodiments

### [Method for detecting risk of colorectal cancer]

In one embodiment, the present invention provides a method as defined in the claims, for determining the risk of colorectal cancer, where the method includes a step of detecting a loss-of-function mutation of a gene involved in IL-17 signaling in a colorectal epithelial tissue sample derived from a subject, in which the presence of the loss-of-function mutation indicates that the subject has a high risk of colorectal cancer. In the method of the present embodiment, the subject is a patient with ulcerative colitis. In addition, the colorectal epithelial tissue sample is a sample of an inflamed region.

It can also be said that the method of the present embodiment is a method for collecting data for determining the risk of colorectal cancer. That is, it can be also said that the method of the present embodiment is a method for collecting data for determining the risk of colorectal cancer, including a step of detecting a loss-of-function mutation of a gene involved in IL-17 signaling in a colorectal epithelial tissue sample derived from a subject, in which the presence of the loss-of-function mutation is data indicating that the subject has a high risk of colorectal cancer.

As will be described later in Examples, the inventors have revealed that in the inflamed region of a patient with ulcerative colitis, a loss-of-function mutation of a gene involved in IL-17 signaling is frequently present.

Further, the inventors have revealed that a patient with ulcerative colitis who has a loss-of-function mutation of a gene involved in IL-17 signaling in the colorectal epithelial tissue is often associated with a high risk for colitis-associated tumor.

As a result, the presence of a loss-of-function mutation of a gene involved in IL-17 signaling in a colorectal epithelial tissue derived from a subject indicates that the subject has a high risk of colorectal cancer. In the present specification, the description that a subject has a high risk of cancer means that, for example, that the subject is highly likely to develop a colitis-associated tumor in the near future, that the subject is highly likely to already have a benign or malignant tumor, or that a colitis-associated tumor already developed in the subject is highly likely to become malignant.

In the method of the present embodiment, the gene involved in IL-17 signaling include a PIGR gene, an NFKBIZ gene, a TRAF3IP2 gene, an IL17RA gene, a ZC3H12A gene, and a NOS2 gene.

The NCBI accession number of the cDNA of the PIGR gene is NM_002644.3 or the like. In the present specification, the protein encoded by the PIGR gene may be referred to as a PIGR protein. The NCBI accession number of the PIGR protein is NP_002635.2 or the like.

The NCBI accession number of the cDNA of the NFKBIZ gene is NM_001005474.2, NM_031419.3, or the like. In the present specification, the protein encoded by the NFKBIZ gene may be referred to as an NFKBIZ protein. The NCBI accession number of the NFKBIZ protein is NP_113607.1, NP_001005474.1, or the like.

The NCBI accession number of the cDNA of the TRAF3IP2 gene is NM_001164281.2, NM_001164283.2, NM_147200.2, NM_147686.3, NM_001164282.1, or the like. In the present specification, the protein encoded by the TRAF3IP2 gene may be referred to as a TRAF3IP2 protein. The NCBI accession number of the TRAF3IP2 protein is NP_671733.2, NP_001157753.1, NP_679211.2, or the like.

The NCBI accession number of the cDNA of the IL17RA gene is NM_001289905.1, NM_014339.6, or the like. In the present specification, the protein encoded by the IL17RA gene may be referred to as an IL17RA protein. The NCBI accession number for the IL17RA protein is NP_001276834.1, NP_055154.3, or the like.

The NCBI accession number of the cDNA of the ZC3H12A gene is NM_001323550.1, NM_001323551.1, NM_025079.2, or the like. In the present specification, the protein encoded by the ZC3H12A gene may be referred to as a ZC3H12A protein. The NCBI accession number of the ZC3H12A protein is NP_079355.2, NP_001310480.1, NP_001310479.1, or the like.

The NCBI accession number of the cDNA of the NOS2 gene is NM_000625.4, NM_153292.1, or the like. In the present specification, the protein encoded by the NOS2 gene may be referred to as a NOS2 protein. The NCBI accession number of the NOS2 protein is NP_000616.3 or the like.

The IL-17 (IL-17A) receptor consists of a heterodimer of an IL17RA protein and an IL17RC protein. The TRAF3IP2 protein (the Act1 protein) is an adapter of the IL-17 receptor and is essential for the downstream activation of the NF-κB signal.

The NFKBIZ protein (the IκBζ protein) is activated by the NF-κB signal activation and activates NF-κB by the positive feedback control. IL-17 stimulation upregulates the expression of the NFKBIZ protein, the PIGR protein, and the like.

For this reason, it can be said that the gene involved in IL-17 signaling is a gene involved in the expression of the PIGR protein.

IgA, which is one kind of immunoglobulin, is secreted from a large number of plasma cells present in the lamina propria under the mucosa, binds to the PIGR protein expressed on the cell membrane on the basal membrane side of the mucosal epithelial cell with a J chain, and is directly incorporated into the epithelial cell together with PIGR protein. Subsequently, the incorporated IgA-PIGR complex crosses inside the cell (transcytosis), and a part (a secretary component, SC) of the PIGR protein is cleaved on the surface side of the mucosa. As a result, an IgA-SC protein complex (a secretory IgA) is released to the surface side of the mucosa. It can be said that the secretory IgA is formed by further binding the SC protein (the secretory component) to the dimeric IgA bound by the J chain.

The J chain is a protein encoded by a Joining Chain Of Multimeric IgA And IgM (JCHAIN) gene. The NCBI accession number of the cDNA of the JCHAIN gene is NM_144646.3 or the like. In the present specification, the protein encoded by the JCHAIN gene may be referred to as a JCHAIN protein, a J chain, or the like. The NCBI accession number of the JCHAIN protein is NP_653247.1 or the like.

In the method of the present embodiment, the loss-of-function mutation may be detected in any one of the genes involved in IL-17 signaling described above, or the loss-of-function mutation may be detected in two or more thereof. In a case where the loss-of-function mutation is present in these genes, a subject can be determined to have a high risk of colorectal cancer.

In addition, the loss-of-function mutation may be present in one allele or both alleles; however, in a case where the loss-of-function mutation is present in both alleles, it can be determined that a subject has a high risk of colorectal cancer as compared with a case where the subject has the loss-of-function mutation in one allele.

In addition, it can be determined that the larger the number of genes having the loss-of-function mutation is, the higher risk of colorectal cancer the subject has.

The loss-of-function mutation is not particularly limited as long as it is a mutation by which a functional protein involved in IL-17 signaling is not expressed, and examples thereof include a truncating mutation (a short type mutation). Examples of the truncating mutation include a nonsense mutation, a frameshift mutation, and a mutation at a splicing site.

The step of detecting a loss-of-function mutation may be carried out by sequencing genes involved in IL-17 signaling, that is, mRNA or genomic DNA of the PIGR gene, the NFKBIZ gene, the TRAF3IP2 gene, the IL17RA gene, the ZC3H12A gene, the NOS2 gene, and the like. Alternatively, it may be carried out by immunostaining of a PIGR protein, a secretory IgA protein, an NFKBIZ protein, a TRAF3IP2 protein, an IL17RA protein, a ZC3H12A protein, or a NOS2 protein in a colorectal epithelial tissue sample derived from a subject.

In a case where the step of detecting a loss-of-function mutation is carried out by immunostaining, the immunostaining may be carried out on any one of the PIGR protein, the secretory IgA protein, the NFKBIZ protein, the TRAF3IP2 protein, the IL17RA protein, the ZC3H12A protein, and the NOS2 protein, or the immunostaining may be carried out on two or more kinds thereof. Among the above, the immunostaining of the PIGR protein or the secretory IgA protein is preferable.

The immunostaining of the secretory IgA protein may be carried out by staining with an anti-IgA antibody or by staining with an antibody (an anti-SC antibody) against the SC protein (the secretory component) which is a part of the PIGR protein. Alternatively, it may be carried out by staining with an antibody against the J chain (the anti-J chain antibody) that binds IgA molecules to each other to form a dimeric IgA molecule.

As will be described later in Examples, in a case where the presence of cells in which the expression of the PIGR protein or the secretory IgA protein is not observed is detected in the colorectal epithelial tissue sample as a result of the immunostaining, it can be determined that a loss-of-function mutation of a gene involved in the expression of the PIGR protein is present.

More specifically, in a case where an intestinal crypt in which the expression of the PIGR protein has disappeared is detected as a result of the immunostaining, or in a case where an intestinal crypt in which the IgA (secretory IgA) protein in the cytoplasm has disappeared is detected, it can be determined that a loss-of-function mutation of a gene involved in the expression of the PIGR protein is present.

### [Kit]

In one embodiment, the present invention provides a kit for determining the risk of colorectal cancer, consisting of probes for detecting loss-of-function mutation in all the genes of the group consisting of a PIGR gene, an NFKBIZ gene, a TRAF3IP2 gene, an IL17RA gene, a ZC3H12A gene, and a NOS2 gene, or, consisting of primers for amplifying all the genes of the group consisting of a PIGR gene, an NFKBIZ gene, a TRAF3IP2 gene, an IL17RA gene, a ZC3H12A gene, and a NOS2 gene, or consisting of specific binding substances to all the proteins of the group consisting of a PIGR protein, a secretory IgA protein, an NFKBIZ protein, a TRAF3IP2 protein, an IL17RA protein, a ZC3H12A protein, and a NOS2 protein.

With the kit of the present embodiment, the above-described method for determining the risk of colorectal cancer can be executed.

In a case where the kit of the present embodiment includes a probe, the probe is not particularly limited as long as the probe specifically hybridizes to the mRNA or genomic DNA of the PIGR gene, the NFKBIZ gene, the TRAF3IP2 gene, the IL17RA gene, the ZC3H12A gene, or the NOS2 gene, and thus the sequencing or the identification of mutations is possible. The probe is preferably immobilized on a carrier to form a DNA microarray.

As a result of sequencing or identifying mutations by hybridizing the mRNA or genomic DNA of these genes to the probe, in a case where a mutation by which a functional protein involved in IL-17 signaling is not expressed is present, it can be determined that a loss-of- function mutation of a gene involved in IL-17 signaling is present.

In a case where the kit of the present embodiment includes a primer, the primer is used to carry out a PCR reaction or the like, whereby the mRNA or genomic DNA of the PIGR gene, the NFKBIZ gene, the TRAF3IP2 gene, the IL17RA gene, the C3H12A gene, or the NOS2 gene is amplified, and subsequently, the base sequence of the obtained amplification product is obtained by sequencing.

As a result of the sequencing, in a case where a mutation by which a functional protein involved in IL-17 signaling is not expressed is present, it can be determined that a loss-of-function mutation of a gene involved in IL-17 signaling is present.

Examples of the mutation by which a functional protein involved in IL-17 signaling is not expressed include a truncating mutation (a short type mutation). Examples of the truncating mutation include a nonsense mutation, a frameshift mutation, and a mutation at a splicing site.

In a case where the kit of the present embodiment includes a specific binding substance to a PIGR protein, a secretory IgA protein, an NFKBIZ protein, a TRAF3IP2 protein, an IL17RA protein, a ZC3H12A protein, or a NOS2 protein, a colorectal epithelial tissue sample derived from a subject is immunostained using the specific binding substance.

As a result of the immunostaining, in a case where the presence of cells in which the expression of the PIGR protein or the secretory IgA protein is not observed is detected in the colorectal epithelial tissue sample as a result of the immunostaining, it can be determined that a loss-of-function mutation of a gene involved in the expression of the PIGR protein is present.

As described above, the specific binding substance to the secretory IgA protein may be a specific binding substance to IgA, may be a specific binding substance to the SC protein (the secretory component), or may be a specific binding substance to the J chain.

Examples of the specific binding substance include an antibody, an antibody fragment, and an aptamer. The antibody may be prepared by immunizing an animal such as a mouse or may be prepared by screening an antibody library such as a phage library. Examples of the antibody fragment include F(ab')₂, Fab', Fab, Fv, and scFv. The aptamer is not particularly limited as long as it has a specific binding ability to a target substance, and examples thereof include a nucleic acid aptamer and a peptide aptamer.

### [Other Embodiments]

Disclosed, but not claimed, is a method for treating colorectal cancer, including a step of detecting a loss-of-function mutation of a gene involved in IL-17 signaling in a colorectal epithelial tissue sample derived from a subject, and a step of administering a therapeutic agent and/or an anticancer agent for ulcerative colitis to a subject in a case where the loss-of-function mutation is present.

In the present embodiment, examples of the subject include a patient with ulcerative colitis. In addition, the gene involved in IL-17 signaling and the loss-of-function mutation of a gene are the same as those described above.

In addition, examples of the anticancer agent include, but are not limited to, cell-killing anticancer agents such as oxaliplatin, irinotecan, 5-fluorouracil, and capecitabin; and molecular-targeted anticancer agents such as bevacizumab, ramucirumab, aflibercept, cetuximab, and panitumumab. In addition, leucovorin may be administered together with the above-described cell-killing anticancer agent.

### Examples

Next, the present invention will be described in more detail by showing Examples, but the present invention is not limited to Examples below.

### [Experimental Example 1]

### (Preparation of organoid)

The experiment was carried out based on the approval of the Keio University School of Medicine Ethics Committee (approval numbers 20120057, 20130512, and G3553-(7)). Intestinal tissue samples were collected from patients with ulcerative colitis who received informed consent and underwent colonoscopy or surgery.

In a case of an intestinal tissue sample derived from a healthy subject, the stromal tissue was removed from the intestinal tissue, and the epithelial tissue was cut to a size of about 1 mm³. Epithelial tissue fragments were washed at least 5 times with phosphate buffered saline (PBS) ice-cooled. Subsequently, intestinal crypts were released by treatment at 4°C for 1 hour while gently stirring in 2.5 mM EDTA.

Subsequently, the released intestinal crypts were suspended in Matrigel (registered trade name, BD Biosciences), and aliquots of 25 µL as a droplet were seeded on a 48-well plate. After the Matrigel got gelled, a medium having a composition shown in Table 1 was overlayered.

**[Table 1]**

| |
|---|
| Advanced DMEM/F-12 medium (Thermo Fisher Scientific, Inc.) |
| Penicillin/Streptomycin (Thermo Fisher Scientific, Inc.) |
| 10 mM HEPES (Thermo Fisher Scientific, Inc.) |
| 2 mM GlutaMAX (Thermo Fisher Scientific, Inc.) |
| 1 x B-27 Supplement (Thermo Fisher Scientific, Inc.) |
| 10 nM gastrin I (Sigma-Aldrich Co., LLC) |
| 1 mM N-acetyl cysteine (Sigma-Aldrich Co., LLC) |
| 100 ng/mL recombinant mouse Noggin (PeproTech) |
| 50 ng/mL recombinant mouse EGF (Thermo Fisher Scientific, Inc.) |
| 100 mg/mL recombinant human IGF-1 (BioLegend) |
| 50 ng/mL recombinant human FGF-2 (PeproTech) |
| 1 µg/mL recombinant human R-Spondin 1 (R&D Systems Inc.) |
| 500 nM A83-01 (Tocris Bioscience) |
| 20% Afamin-Wnt-3A serum-free conditioned medium |

A sample of a non-inflamed region, derived from a patient with ulcerative colitis (hereinafter, may be referred to as "UC^{uninf}"), a sample of an inflamed region, derived from a patient with ulcerative colitis (hereinafter, may be referred to as "UC^{inf}"), and a fragmented mucosa tissue in a case of a sample of a colitis-associated tumor, derived from a patient with ulcerative colitis (hereinafter, may be referred to as "CAN") were digested using Liberase ("Liberase TH, Research Grade", Roche) at 37°C for 1 hour. Subsequently, the remaining fragments were digested using TrypLE Express (Thermo Fisher Scientific, Inc.) at 37°C for 20 minutes. Subsequently, the digested tissue was embedded in Matrigel, and the above-described medium was overlayered.

The medium was replaced with a new medium every two days. In addition, the formed organoids were passaged once a week using TrypLE Express at a split ratio of 1:10. For two days after the establishment of the organoid and two days after the passage, 10 µM Y-27632 (FUJIFILM Wako Pure Chemical Corporation) was added to the medium.

As a result, 122 normal organoid strains (clones) derived from 45 patients with ulcerative colitis and 13 healthy subjects were obtained. Among the 45 patients with ulcerative colitis, 22 patients had a colitis-associated tumor. Then, organoids including 19 strains of organoids derived from the colitis-associated tumors, including 5 strains having no corresponding normal organoids, were obtained.

Hereinafter, an organoid derived from a non-inflamed region derived from a patient with ulcerative colitis may be referred to as a "UC^{uninf} organoid". In addition, an organoid derived from an inflamed region derived from a patient with ulcerative colitis may be referred to as a "UC^{inf} organoid". In addition, an organoid derived from a healthy subject may be referred to as an "HC organoid". In addition, an organoid derived from a colitis-associated tumor may be referred to as a "CAN organoid".

### [Experimental Example 2]

### (Examination 1 of loss-of-function mutation of gene involved in IL-17 signaling)

Whole exome sequencing of each organoid clone established in Experimental Example 1 was carried out to examine whether gene mutations specific to the UC^{inf} organoid were accumulated or not. The inventors focused on the truncating mutations (the short type mutations) including a nonsense mutation, a frameshift mutation, and a mutation at a splicing site, which may cause a loss-of-function mutation.

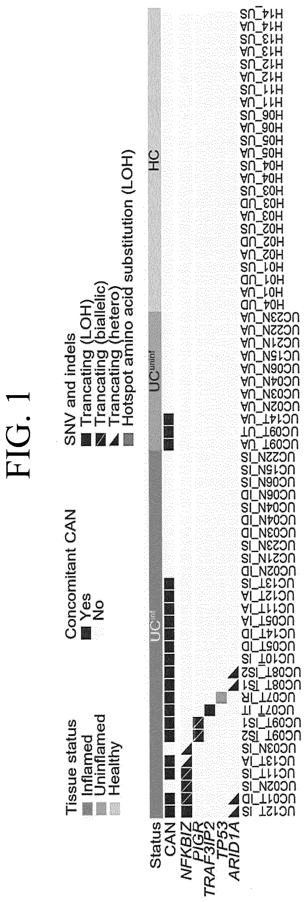

Fig. 1 is a diagram showing the analysis results. In Fig. 1, the horizontal axis indicates the clone name of each organoid clone. In addition, "UC^{inf}" indicates a case of being a UC^{inf} organoid, "UC^{uninf}" indicates a case of being a UC^{uninf} organoid, and "HC" indicates a case of being an HC organoid. In addition, in "CAN", a black square indicates that a patient with ulcerative colitis has a colitis-associated tumor. In addition, "Truncating" indicates a truncating mutation.

In addition, "NFKBIZ" indicates the NFKBIZ gene, "PIGR" indicates the PIGR gene, "TRAF3IP2" indicates the TRAF3IP2 gene, "TP53" indicates the TP53 gene, and "ARID1A" indicates the ARID1A gene.

In addition, a black square indicates a case of having a loss-of-heterozygousity (LOH), a black triangle indicates a case of having a truncating mutation in one allele, a black square with white diagonal lines indicates a case of having a truncating mutation in both alleles, and "Hotspot amino substitution" indicates a case of having an amino acid substitution mutation in the hotspot.

From the analysis results, it was revealed that in the UC^{inf} organoid, truncating mutations repeatedly occur in the NFKBIZ gene and the PIGR gene involved in the immune function in the mucosa.

In addition, five strains of UC^{inf} organoids had truncating mutations in the NFKBIZ gene or the PIGR gene in both alleles, which differed in mutation pattern from each other. This result indicates that specific selection is made in the inflammatory environment of ulcerative colitis.

It was also revealed that the truncating mutations in the NFKBIZ gene and the TRAF3IP2 gene are accompanied by a loss-of-heterozygosity (LOH) in two strains of UC^{inf} organoids.

The loss-of-heterozygosity (LOH) was rare in the HC organoids, whereas the loss-of-heterozygosity (LOH) was detected in 7 strains among 29 strains of UC^{inf} organoids (data not shown) as a result of the single nucleotide polymorphism analysis of the whole exome sequencing data. This result further supports that specific selection is made in the inflammatory environment of ulcerative colitis.

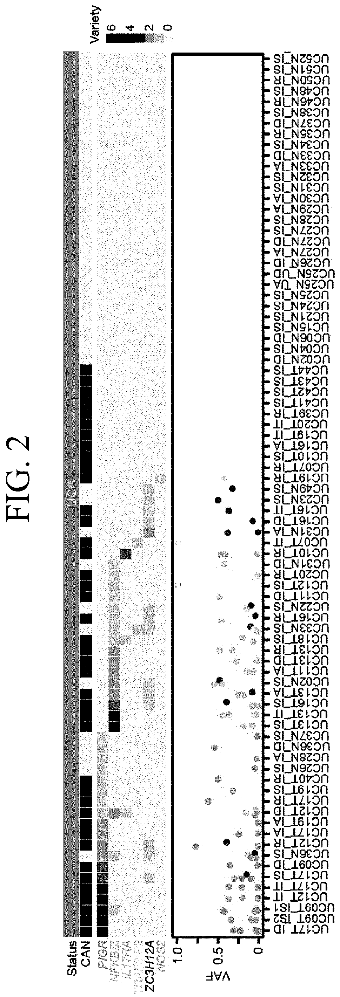

Fig. 2 is a diagram showing the results of carrying out analysis by increasing the number of examples of the UC^{inf} organoid. In Fig. 2, the horizontal axis indicates the clone name of each organoid clone. In addition, "UC^{inf}" indicates a case of being a UC^{inf} organoid. In addition, "PIGR" indicates the PIGR gene, "NFKBIZ" indicates the NFKBIZ gene, "IL17RA" indicates the IL17RA gene, "TRAF3IP2" indicates the TRAF3IP2 gene, "ZC3H12A" indicates the ZC3H12A gene, and "NOS2" indicates the NOS2 gene. In addition, in "CAN", a black square indicates that a patient with ulcerative colitis has a colitis-associated tumor.

In addition, a square indicates the presence of truncating mutation, and the density of the square corresponds to the number of different mutants present in each gene. In addition, "VAF" indicates a Variant allele fraction, and each dot indicates the proportion of mutated allele reads with respect to all of the reads.

From the analysis results, it was confirmed that truncating mutations frequently occur in the PIGR gene and the NFKBIZ gene. In addition, truncating mutations were found in the IL17RA gene, TRAF3IP2 gene, ZC3H12A gene, and NOS2 gene. This result further supports the accumulation of truncating mutations in genes involved in IL-17 signaling in UC^{inf} organoids.

### [Experimental Example 3]

### (Examination 2 of loss-of- function mutation of gene involved in IL-17 signaling)

In Experimental Example 2, the accumulation of truncating mutations was examined using an organoid clone. In the present Experimental Example, genomic DNA was extracted from a UC^{inf} organoid, a UC^{uninf} organoid, and an HC organoid, which were polyclonal and had been cultured in less than 4 passages, and target genes were sequenced to examine the accumulation of truncating mutations. As the target genes, 37 genes including genes involved in IL-17 signaling and driver genes for sporadic colorectal cancer were examined.

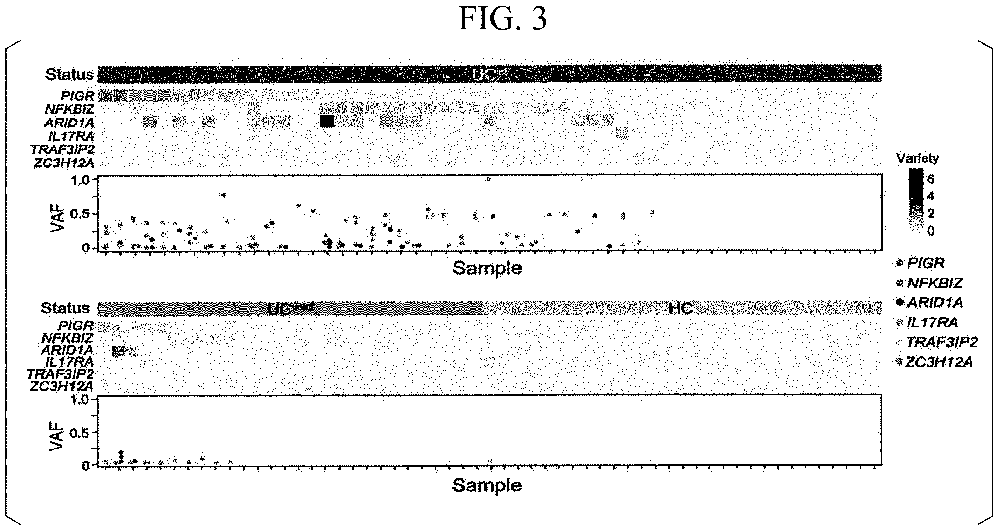

Fig. 3 is a diagram showing the analysis results. In Fig. 3, the horizontal axis indicates the results of experiments carried out independently. In addition, "UC^{inf}" indicates a case of being a UC^{inf} organoid, "UC^{uninf}" indicates a case of being a UC^{uninf} organoid, and "HC" indicates a case of being an HC organoid.

In addition, "PIGR" indicates the PIGR gene, "NFKBIZ" indicates the NFKBIZ gene, "ARID1A" indicates the ARID1A gene, "IL17RA" indicates the IL17RA gene, "TRAF3IP2" indicates the TRAF3IP2 gene, and "ZC3H12A" indicates the ZC3H12A gene.

In addition, a square indicates the presence of truncating mutation, and the density of the square corresponds to the number of different mutants present in each gene. In addition, "VAF" indicates a Variant allele fraction, and each dot indicates the proportion of mutated allele reads with respect to all of the reads.

From the analysis results, it was confirmed that truncating mutations frequently occur in the NFKBIZ gene and the PIGR gene. In addition, a truncating mutation of the IL17RA gene, which was not detected in Experimental Example 2, was found in the four UC^{inf} organoids. This result further supports the accumulation of truncating mutations in genes involved in IL-17 signaling in UC^{inf} organoids.

### [Experimental Example 4]

### (Immunostaining 1 of PIGR protein)

In Experimental Examples 2 and 3, it was revealed that the truncating mutation of the PIGR gene frequently occurs in the UC^{inf} organoid. In the present Experimental Example, thin slices of a colorectal epithelial tissue sample of an inflamed region, derived from a patient with ulcerative colitis, were immunostained to examine the expression of PIGR protein.

For comparison, thin slices of a colorectal epithelial tissue sample derived from a healthy subject were immunostained to examine the expression of PIGR protein. The same examination was also carried out on the colorectal epithelial tissue sample of the non-inflamed region, derived from a patient with ulcerative colitis.

In addition, in some cases, immunostaining of the secretory IgA protein and the cytokeratin 20 (CK20) protein was also carried out. Here, CK20 is a marker for glandular epithelial cells and is stained to confirm the retention of protein antigens in the sample during immunostaining. An anti-PIGR antibody (catalog number "HPA012012", Sigma-Aldrich Co., LLC) was used for staining the PIGR protein. An anti-SC antibody (catalog number "ab3924", Abcam plc) was used for staining the secretory IgA protein. An anti-CK20 antibody (catalog number "M7019", Agilent Technologies, Inc.) was used for staining the CK20 protein.

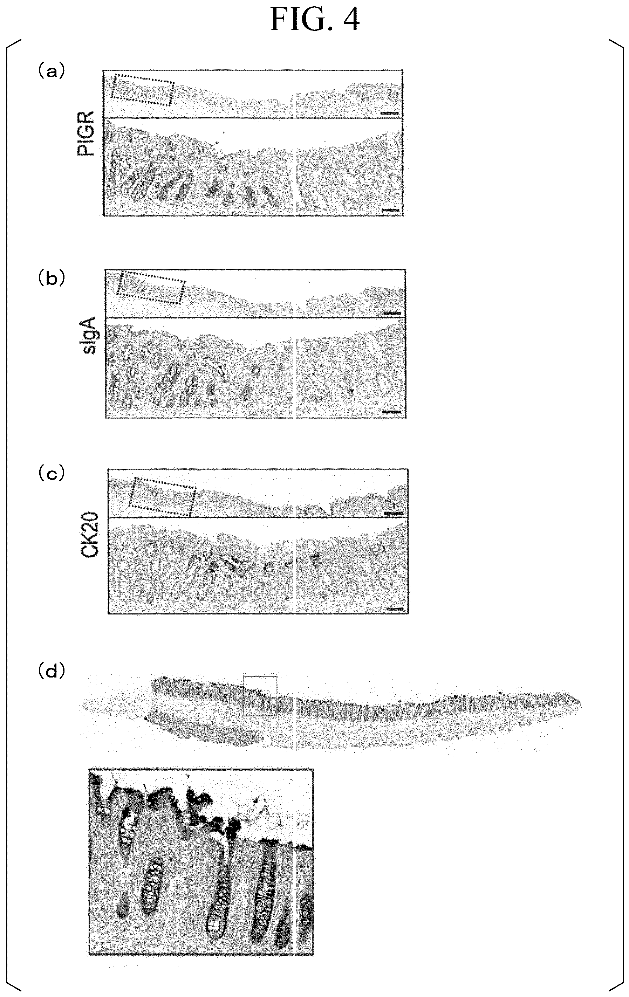

Figs. 4 (a) to (c) are typical photomicrographs showing the results of immunostaining of a colorectal epithelial tissue sample derived from a patient with ulcerative colitis. Fig. 4 (a) is the result of detecting the PIGR protein, Fig. 4 (b) is the result of detecting the secretory IgA protein, and Fig. 4 (c) is the result of detecting the CK20 protein.

In Figs. 4 (a) to 4 (c), the upper part shows a low-magnification photomicrograph, and the lower part shows a high-magnification photomicrograph in which the area surrounded by a square in the low-magnification photomicrograph is enlarged. The scale bar of the low-magnification photomicrograph is 500 µm, and the scale bar of the high-magnification photomicrograph is 100 µm.

In addition, Fig. 4 (d) is a photomicrograph showing the result of detecting the expression of the PIGR protein by immunostaining of the colorectal epithelial tissue sample of the colorectal epithelial tissue sample derived from a healthy subject. In Fig. 4 (d), the upper part shows a low-magnification photomicrograph, and the lower part shows a high-magnification photomicrograph in which the area surrounded by a square in the low-magnification photomicrograph is enlarged.

From the immunostaining results, as shown in Fig. 4(a), it was observed that the PIGR protein was ubiquitous in the colorectal epithelial tissue sample derived from a healthy subject and the colorectal epithelial tissue in the non-inflamed region derived from a patient with ulcerative colitis, whereas a cluster of intestinal crypts in which the expression of the PIGR protein was disappeared was observed in the colorectal epithelial tissue in the inflamed region, derived from a patient with ulcerative colitis. This result was conceived to be due to the truncating mutation in the PIGR gene.

Further, as shown in Fig. 4 (b), in the intestinal crypt where the expression of PIGR protein was disappeared, the presence of the secretory IgA protein in the cytoplasm was also disappeared. This result further supports a loss-of-function mutation occurring in the PIGR gene in the inflamed region derived from a patient with ulcerative colitis.

### [Experimental Example 5]

### (Examination 3 of loss-of-function mutation of gene involved in IL-17 signaling)

Whole exome sequencing of a clone of an organoid derived from a colitis-associated tumor (hereinafter, may be referred to as a "CAN" organoid), established in Experimental Example 1, was carried out to examine whether gene mutations specific to the NFKBIZ gene, the PIGR gene, theTRAF3IP2 gene, the TP53 gene, and the ARID1A gene, which had been examined in Experimental Example 2, were accumulated or not.

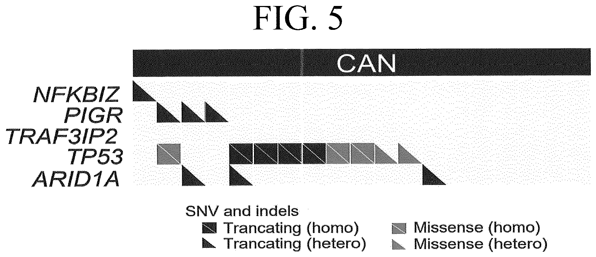

Fig. 5 is a diagram showing the analysis results. In Fig. 5, the horizontal axis indicates each organoid clone. Further, "CAN" indicates a case of being a CAN organoid. In addition, "NFKBIZ" indicates the NFKBIZ gene, "PIGR" indicates the PIGR gene, "TRAF3IP2" indicates the TRAF3IP2 gene, "TP53" indicates the TP53 gene, and "ARID1A" indicates the ARID1A gene.

In addition, a black triangle indicates a case of having a truncating mutation in one allele, a black square with white diagonal lines indicates a case of having a truncating mutation in both alleles, a gray triangle indicates a case of having a missense mutation in one allele, and a gray square with white diagonal lines indicates a case of having a missense mutation in both alleles.

As a result of the examination, it was revealed that the truncating mutations of the NFKBIZ gene and the PIGR gene are rare in the CAN organoids and that the mutation is heterozygous even in a case of being present. This result suggests that the truncating mutations in the NFKBIZ gene and the PIGR gene do not directly contribute to colitis-related carcinogenesis.

### Industrial Applicability

According to the present invention, it is possible to provide a technique for determining the risk of colorectal cancer in a patient with ulcerative colitis.

## Claims

1. A method for determining a risk of colorectal cancer of a patient with ulcerative colitis, comprising:
a step of detecting a loss-of-function mutation of a gene involved in interleukin (IL)-17 signaling in a colorectal epithelial tissue sample of an inflamed and non-cancer region derived from the patient,
wherein the gene involved in IL-17 signaling is a PIGR gene, an NFKB Inhibitor Zeta (NFKBIZ) gene, a TRAF3 Interacting Protein 2 (TRAF3IP2) gene, an Interleukin 17 Receptor A (IL17RA) gene, a Zinc Finger CCCH-Type Containing 12A (ZC3H12A) gene, or a Nitric Oxide Synthase 2 (NOS2) gene,
wherein a presence of the loss-of-function mutation indicates that the patient has a high risk of colorectal cancer.

2. The method according to Claim 1,
wherein the step of detecting the loss-of-function mutation of the gene involved in IL-17 signaling is carried out by immunostaining of a PIGR protein, a secretory IgA protein, an NFKBIZ protein, a TRAF3IP2 protein, an IL17RA protein, a ZC3H12A protein, or a NOS2 protein in the colorectal epithelial tissue sample, and
as a result of the immunostaining, a case where a presence of cells in which an expression of the PIGR protein, the secretory IgA protein, the NFKBIZ protein, the TRAF3IP2 protein, the IL17RA protein, the ZC3H12A protein, or the NOS2 protein is not observed is detected in the colorectal epithelial tissue sample indicates that the loss-of-function mutation is present.

3. A kit for determining a risk of colorectal cancer of a patient with ulcerative colitis, consisting of:
probes for detecting loss-of-function mutation of a PIGR gene, an NFKBIZ gene, a TRAF3IP2 gene, an IL17RA gene, a ZC3H12A gene, and a NOS2 gene;
primers for amplifying a PIGR gene, an NFKBIZ gene, a TRAF3IP2 gene, an IL17RA gene, a ZC3H12A gene, and a NOS2 gene; or
specific binding substances to a PIGR protein, a secretory IgA protein, an NFKBIZ protein, a TRAF3IP2 protein, an IL17RA protein, a ZC3H12A protein, and a NOS2 protein.

## Patentansprüche

1. Verfahren zum Bestimmen eines Risikos für ein Kolorektalkarzinom eines Patienten mit Colitis ulcerosa, umfassend:
einen Schritt des Erkennens einer Funktionsverlustmutation eines Gens, das an der Interleukin(IL)-17-Signalisierung beteiligt ist, in einer Probe kolorektalen Epithelgewebes einer entzündeten und Nichtkrebsregion, die von dem Patienten stammt,
wobei das an der IL-17-Signalisierung beteiligte Gen ein PIGR-Gen, ein NFKB-Inhibitor-Zeta(NFKBIZ)-Gen, ein TRAF3-Interacting-Protein-2(TRAF3IP2)-Gen, ein Interleukin-17-Receptor-A(IL17RA)-Gen, ein Zink-Finger-CCCH-Type-Containing-12A(ZC3H12A)-Gen oder ein Stickstoffoxidsynthase-2(NOS2)-Gen ist,
wobei das Vorhandensein der Funktionsverlustmutation darauf hinweist, dass der Patient ein hohes Risiko für ein Kolorektalkarzinom aufweist.

2. Verfahren nach Anspruch 1,
wobei der Schritt des Erkennens der Funktionsverlustmutation des an der IL-17-Signalisierung beteiligten Gens durch Immunfärbung eines PIGR-Proteins, eines sekretorischen IgA-Proteins, eines NFKBIZ-Proteins, eines TRAF3IP2-Proteins, eines IL17RA-Proteins, eines ZC3H12A-Proteins oder eines NOS2-Proteins in der Probe kolorektalen Epithelgewebes durchgeführt wird und
als Ergebnis der Immunfärbung ein Fall, in dem ein Vorhandensein von Zellen, in denen keine Expression des PIGR-Proteins, des sekretorischen IgA-Proteins, des NFKBIZ-Proteins, des TRAF3IP2-Proteins, des IL17RA-Proteins, des ZC3H12A-Proteins oder des NOS2-Proteins beobachtet wird, in der Probe kolorektalen Epithelgewebes erkannt wird, darauf hinweist, dass die Funktionsverlustmutation vorhanden ist.

3. Kit zum Bestimmen eines Risikos für ein Kolorektalkarzinom eines Patienten mit Colitis ulcerosa, bestehend aus:
Sonden zum Erkennen einer Funktionsverlustmutation eines PIGR-Gens, eines NFKBIZ-Gens, eines TRAF3IP2-Gens, eines IL17RA-Gens, eines ZC3H12A-Gens und eines NOS2-Gens;
Primer zum Amplifizieren eines PIGR-Gens, eines NFKBIZ-Gens, eines TRAF3IP2-Gens, eines IL17RA-Gens, eines ZC3H12A-Gens und eines NOS2-Gens; oder
spezifische Bindungssubstanzen an ein PIGR-Protein, ein sekretorisches IgA-Protein, ein NFKBIZ-Protein, ein TRAF3IP2-Protein, ein IL17RA-Protein, ein ZC3H12A-Protein und ein NOS2-Protein.

## Revendications

1. Procédé permettant la détermination d'un risque de cancer colorectal d'un patient atteint de colite ulcéreuse, comprenant :
une étape de détection d'une mutation de perte de fonction d'un gène impliqué dans la signalisation de l'interleukine (IL)-17 dans un échantillon de tissu épithélial colorectal d'une région enflammée et non cancéreuse prélevé chez le patient,
ledit gène impliqué dans la signalisation de l'IL-17 étant un gène PIGR, un gène NFKBIZ (NFKB Inhibitor Zeta), un gène TRAF3IP2 (TRAF3 Interacting Protein 2), un gène IL17RA (Interleukin 17 Receptor A), un gène ZC3H12A (Zinc Finger CCCH-Type Containing 12A), ou un gène NOS2 (Nitric Oxide Synthase 2),
une présence de la mutation de perte de fonction indiquant que le patient présente un risque élevé de cancer colorectal.

2. Procédé selon la revendication 1,
ladite étape de détection de la mutation de perte de fonction du gène impliqué dans la signalisation de l'IL-17 étant effectuée par immunocoloration d'une protéine PIGR, d'une protéine IgA sécrétoire, d'une protéine NFKBIZ, d'une protéine TRAF3IP2, d'une protéine IL17RA, d'une protéine ZC3H12A ou d'une protéine NOS2 dans l'échantillon de tissu épithélial colorectal, et
à la suite de l'immunocoloration, un cas où une présence de cellules dans lesquelles une expression de la protéine PIGR, de la protéine IgA sécrétoire, de la protéine NFKBIZ, de la protéine TRAF3IP2, de la protéine IL17RA, de la protéine ZC3H12A ou de la protéine NOS2 n'est pas observée est détecté dans l'échantillon de tissu épithélial colorectal indiquant que la mutation de perte de fonction est présente.

3. Kit permettant la détermination d'un risque de cancer colorectal d'un patient atteint de colite ulcéreuse, constitué :
de sondes destinées à détecter une mutation de perte de fonction d'un gène PIGR, d'un gène NFKBIZ, d'un gène TRAF3IP2, d'un gène IL17RA, d'un gène ZC3H12A et d'un gène NOS2 ;
d'amorces destinées à amplifier un gène PIGR, un gène NFKBIZ, un gène TRAF3IP2, un gène IL17RA, un gène ZC3H12A et un gène NOS2 ; ou
de substances de liaison spécifiques à une protéine PIGR, une protéine IgA sécrétoire, une protéine NFKBIZ, une protéine TRAF3IP2, une protéine IL17RA, une protéine ZC3H12A et une protéine NOS2.
